# EUROPÄISCHE PATENTSCHRIFT

(11) **EP 3 114 208 B1**
(45) Veröffentlichungstag und Bekanntmachung des Hinweises auf die Patenterteilung: **26.02.2020**
(21) Anmeldenummer: 15712045.2
(22) Anmeldetag: 03.03.2015
(51) Int. Cl.: C12M 1/107, C12M 1/00, C12P 5/02

(54) **VORRICHTUNG UND VERFAHREN ZUR ERZEUGUNG EINER BIOMASSEZUBEREITUNG**
DEVICE AND METHOD FOR GENERATING A BIOMASS PREPARATION
DISPOSITIF ET PROCÉDÉ POUR PRODUIRE UNE PRÉPARATION DE BIOMASSE

(30) Priorität: 03.03.2014 DE 102014003159; 03.03.2014 DE 202014004906 U
(43) Veröffentlichungstag der Anmeldung: 11.01.2017
(73) Patentinhaber: MWK Bionik GmbH, 83119 Obing (DE)
(72) Erfinder: WACKERBAUER, Matthias, 83119 Obing (DE)
(74) Vertreter: Stolmár & Partner Patentanwälte PartG mbB
(86) Internationale Anmeldenummer: PCT/EP2015/000482
(87) Internationale Veröffentlichungsnummer: WO 2015/132000

(56) Entgegenhaltungen:
- WO-A2-2014/095669
- DE-A1-102008 024 388
- DE-A1-102011 118 067
- DE-B3-102011 113 646

## Beschreibung

### Gebiet der Erfindung

Die vorliegende Erfindung betrifft eine Vorrichtung und ein Verfahren zur Erzeugung einer Biomassezubereitung (Biomassezusammensetzung), insbesondere einer fermentierbaren Biomassezubereitung, durch eine Kombination biologischer, mechanischer und / oder thermischer Aktivierung von mikrobiologischen Aktivatoren, die in der Lage sind, schwer aufschließbare biologische Edukte, insbesondere den Ligninanteil von Biomasse, aufzuschließen und vergärbar zu machen.

### Stand der Technik

Herkömmliche Biogasanlagen arbeiten mit hydrolysierenden und methanisierenden Bakterien, die zuckerhaltige Stoffe und andere leicht zu vergärende Kohlenstoffverbindungen zu Methan verstoffwechseln können. Allgemein gesprochen ist Biogas ein aus organischen Substraten mittels anaerober Gär- und Fäulnisprozesse entstandenes Gasgemisch. Es besteht hauptsächlich aus Methan (ca. 50- 80 Vol% in Abhängigkeit vom Einsatzstoff) Kohlendioxid (19 - 49 Vol%) und Wasserdampf (1-4 Vol%) Hauptenergieträger im Biogas. Es wird bei der Verbrennung z. B. in Energie, Sauerstoff und Kohlendioxid umgesetzt.

Biogas-Systeme des Standes der Technik haben jedoch den Nachteil, dass sie insbesondere ligninhaltige Einsatzstoffe wie z. B. Stroh nicht oder fast nicht bzw. nur sehr langsam vergären. Angesichts der aus wirtschaftlichen Gründen endlichen Verweilzeit der Biomassen im Fermenter bleiben folglich bei herkömmlichen Biogasanlagen bis zu mehr als 50 % der Biomassen unvergoren. Dies bedeutet eine erhebliche Ineffizienz und führt zu hohem Einsatzstoffverbrauch. In Zeiten hoher Einsatzstoffpreise ist dies wirtschaftlich sehr nachteilig und gefährdet die Rentabilität der herkömmlichen Biogasanlagen.

Es wurden auch thermophile Biogas-Reaktoren entwickelt, die bei höheren Temperaturen vergären. Dadurch werden der Aufschluss und die Methanisierung der schlechter vergärbaren Stoffanteile etwas besser. Doch immer noch bleiben erhebliche Anteile der Biomassen unvergoren. Insbesondere strohige Biomassen führen zur Ausbildung von Schwimmschichten im Endlager, auch wenn die Gesamtausbeute bereits über 50 % des Kohlenstoffeintrags beträgt. Pflanzliche Biomasse besteht insbesondere aus Cellulose, Hemizellulose und Lignin. Lignozellulose ist durch eine Ligninschicht geschützt und kann deshalb ohne weitere Vorbehandlung nicht oder nur sehr langsam fermentiert werden.

In den vergangenen Jahren wurden zahlreiche Systeme entwickelt, um durch eine Zerkleinerung der Einsatzstoffe eine bessere Vergärung zu erreichen. Diese Systeme haben jedoch einen hohen Eigenenergieverbrauch, insbesondere Stromverbrauch für den mechanischen Antrieb. Folglich ist die so erreichbare Effizienzsteigerung gering bzw. oft sogar fraglich. Zudem gelingt es auch damit nicht in ausreichendem Maße, den Ligninanteil aufzuschließen.

Ebenfalls wurden biologisch und biochemische Substanzen und Verfahren zur Optimierung des Gärungs-Stoffwechsels in den Biogasanlagen entwickelt. Diese Methoden schaffen Abhilfe bei Mangelerscheinungen im Stoffwechsel der Mikroben und können die Ausbeute steigern. Doch ändert auch dies nicht viel an der grundsätzlich schlechten Vergärbarkeit der holzigen und / oder ligninhaltigen Einsatzstoffanteile. Diese Problematik der kaum möglichen Strohvergärung ist umso schwerwiegender, als bis zu 50 % und mehr der Einsatzstoffe wie Maissilage aus strohartiger Biomasse bestehen. Mit herkömmlicher Technik bleibt also typisch ca. 50 % der wertvollen eingesetzten Biomasse unvergoren. Bei Einsatz von stark strohhaltiger Biomasse wie Pferdemist ist eine Vergärung oft sehr schwierig und ineffizient.

Deshalb wurden in den letzten Jahren Aktivierungsverfahren entwickelt, die mit Dampf arbeiten. Das Problem hierbei ist aber, dass Dampf direkt an der Biogasanlage erzeugt werden muss, was einen hohen Aufwand bedeutet. Dabei hat sich herausgestellt, dass auch nach Dampfaufschluss die Gesamteffzienz der Methanisierung nicht ausreichend erhöht werden kann.

Bislang unbefriedigend gelöst ist der möglichst vollständige Aufschluss ligninhaltiger Biomassen wie z. B. Strohanteilen, um auf diese Weise eine sehr viel vollständigere Vergärung der eingesetzten Biomassen zu erreichen. Dabei soll das Verfahren außerdem effizient, sicher und kostensparend sein.

Lignin besteht aus festen Biopolymeren, die in die pflanzliche Zellwand eingelagert werden und dadurch die Verholzung der Pflanzenzelle bewirken. Lignin ist insbesondere für die Druckfestigkeit von Pflanzenbestandteilen verantwortlich, man findet es also im Halm oder Stiel und / oder in den Schalen.

Der natürliche biologische Abbau von Lignin erfolgt nur sehr langsam und ist biologisch komplex. Der Ligninabbau findet dabei immer unter aeroben Bedingungen statt und ist sehr energieintensiv. Er kann entsprechend nicht als alleinige Kohlenstoff- und Energiequelle dienen. Der natürliche Abbau von Lignin kann also nicht für Biomasse-Energieanlagen genutzt werden.

Der technische Ligninabbau spielt vor allem bei der Zellstoffherstellung eine wichtige Rolle. Zur Produktion von Zellstoff muss das Lignin aus der Lignocellulose gelöst und aus dem Prozess entfernt werden. Dabei existieren unterschiedliche Verfahren für den Celluloseaufschluss sowie für die nachfolgende Zellstoffbleiche. In etwa 80 % aller Zellstoffanlagen erfolgt der Aufschluss über das so genannte Sulfatverfahren, auch bekannt als Kraft-Prozess. Eine Alternative stellt der Celluloseaufschluss im Sulfitverfahren dar, bei dem der Ligninabbau durch eine Sulfinierung erfolgt.

Die DE 10 2005 030 895 A1 beschreibt eine Vorrichtung zum Aufschluss von in Dispersion gehaltenem biologischem Material mit einem Gefäß zur Durchleitung des Materials, in dem eine Gruppe von diskreten Schallerzeugern eingebracht ist. Insbesondere mit Ultraschall. Die Vorrichtung soll zur Zerkleinerung der Stücke dienen. Der thermische Energieeintrag durch die Ultraschallerzeuger reicht dabei aus, um die nachgeschaltete Fermentation bei der geeigneten Temperatur erfolgen zu lassen. Die Vorrichtung wird im Zusammenhang mit einem Fermenter und einer Biogasnutzung im Blockheizkraftwerk (BHKW) beschrieben. Die Beschreibung nennt eine Einbringung der vorbereiteten Masse von unten unter hohem Druck eingespritzt, wegen gewollter intensiver Vermischung im Fermenter.

Die DE 10 2011 113 646 B3 beschreibt einen Aufschlussbehälter zur Ballenaufnahme und Zuführung eines vorgeheizten Säure/Wassergemisches über Injektionseinrichtungen sowie Siebelemente im Bodenbereich zur Abtrennung des flüssigen Hydrolysats von der festen Biomasse. Die Aktivierung findet vorzugsweise bei 140-160 °C statt. Verwendet werden mineralische oder organische Säuren. Gelöst werden 50 % des Lignins. Hemicellulose wird zu mehr als 75 in Monosacharide umgewandelt.

Die DE 31 38 309 A1 beschreibt ein Verfahren zum Aufschluss von Holz und anderen ligninhaltigen Substanzen, wobei die im Darmtrakt lebenden Tierchen verwendet werden, wie sie vor allem bei Termiten in Symbiose gefunden werden. Ferner wird genannt, dass die Urtierchen und Bakterien nach ihrer Separierung und Vermehrung tiefgefroren oder gefriergetrocknet werden.

Die DE 10 2011 118 067 A1 beschreibt einen Aktivierungsprozess, wobei Essigsäurebildner zugegeben werden. Der Aktivator setzt Bildung von Essigsäure in Gang, fördert sie und hält die Essigsäurebildung aufrecht. Unter dem Begriff Essigsäure sind auch Acetate zu verstehen, die Salze der Essigsäure. Denn in biochemischen Prozessen liegt größtenteils das Anion CH₃COO⁻ vor. Es bildet sich der sogenannte Essigsäure Acetat Puffer. Es wird mit lebenden Essigsäurebakterien gearbeitet und eine Suspension hergestellt aus Zucker Alkohol und Wasser, die typisch mit 1 Liter pro Kubikmeter Biomasse zugegeben wird. Alternativ wird lebende Hefe zugegeben. Die Umsetzung erfolgt bei einem pH Wert von unter 4. Die Vorrichtung nutzt spiralförmige Rührwerke.

Die DE 198 58 187 C2 beschreibt ein Verfahren und eine Vorrichtung zur Reduzierung der organischen Anteile eines ausfaulbaren Substrats. Dabei wird eine erste anaerobe Faulstufe mit einer thermischen Desintegration kombiniert sowie mit einer nachfolgenden zweiten anaeroben Faulstufe. Trotz des Aufwandes der thermischen Desintegration gelingt jedoch nur der Aufschluss der Hemicellulosen, die Ligninanteile bleiben jedoch unaufgeschlossen. Die Ausbeute an Energie steigt dadurch nur um ca. 10 %.

Alle diese bekannten Verfahren geben zwar an, auch Lignin aufschließen zu können, schaffen dies aber nur in einem sehr geringen und wirtschaftlich nicht gewinnbringenden Umfang. Dies macht einen Ligninabbau in Fermentierungsanlagen bisher technisch und wirtschaftlich ineffizient. Folglich bleiben die ligninhaltigen Pflanzenteile unverwertet und landen bislang im Gärrest (in Biogasanlagen bis zu 50 % der Biomasse).

### Aufgabe der Erfindung

Aufgabe der vorliegenden Erfindung ist es daher, ein Verfahren und eine Vorrichtung zur Erzeugung einer Biomassezubereitung bereitzustellen, wobei auch Biomassen mit sehr hohem Ligninanteil wie z. B. Getreidestroh, Pferdemist, Grünschnitt von Gehölzen, Gärreste und oder Chinaschilf bzw. C4-Gräser als Einsatzstoffe (Edukte) verwendet werden können und dabei der im Edukt enthaltene organische Kohlenstoff möglichst vollständig in energiereiche Kohlenwasserstoffe (z. B. Methan) umwandelt wird.

### Erfindungsgemäße Lösung

Die Aufgabe der vorliegenden Erfindung wird weiter gelöst durch ein Verfahren zur Erzeugung einer Biomassezubereitung wie in Anspruch 1 beschrieben.

Das Verfahren kann in Ausführungsformen weiterhin umfassen:
das Bereitstellen einer Einrichtung zur Druckerhöhung oder Volumenförderung.

Das erfindungsgemäße Verfahren umfasst dabei den optionalen Schritt des Unterwerfens der Kombination aus Edukt und dem Mittel zum Aufschließen des Edukts auf einen Druck P > P_{A} mittels einer Druckerhöhungsvorrichtung, z. B. Pumpe oder Kompressor, z. B. vor oder nach der thermischen Vorrichtung

Das erfindungsgemäße Verfahren ermöglich vorteilhafterweise die besonders effiziente Erzeugung von fermentierbaren Biomassezusammensetzungen, insbesondere den Aufschluß von ligninhaltigen Edukten, wenn auch nicht darauf beschränkt und insbeosnder die Erzeugung von sogenanntem Biogas..

Das erfindungsgemäße Verfahren basiert auf der vorwiegend thermisch aktivierbaren Wirkung eines im wesentlichen mikrobiologischen Aktivators, der das Edukt aufschließt (im folgenden auch Mittel zum Aufschließen des Edukts genannt) die zudem durch Anwendung erhöhter Drücke verstärkt werden kann. Dieser Aktivator unterscheidet sich ganz wesentlich von allen bisher eingesetzten Mitteln.

Der erfindungsgemäße mikrobiologische Aktivator enthält große Mengen von ca. 100 Mrd. Teilchen pro Milliliter an thermophilen druckliebenden Mikroorganismen. Besonders bevorzugte Aktivierungsbedingungen sind Temperaturen zwischen 70 bis 80°C und Drücke zwischen 2 bar und 6 bar.

Der Aktivator wird optional bereits im Vormischer den festen Einsatzstoffen zugegeben. Alternativ ist der Aktivator bereits vorab Bestandteil eines der Einsatzstoffe, die im Einsatzstoffmischer gemischt werden. Der Aktivator wird z. B. dem festen Einsatzstoff wie z. B. Stroh zugegeben, z. B. durch Besprühen aus einer Düsenvorrichtung. Dabei kommt der Aktivator in Kontakt mit der Oberfläche der Einsatzstoffpartikel.

Eine weitere Alternative ist es, den Aktivator direkt in die Rohrleitung in den gemischten Einsatzstoff einzufördern.

Es werden bevorzugt zwei unterschiedliche Gruppen von Mikroorganismen alleine oder bevorzugt in Kombination verwendet:
- Anaerobe oder aerobe Mikroben, die Biomoleküle (z. B. Proteine, Zucker, Fette, Zellulose, Nukleinsäuren) aufbrechen und abbauen, die also z. B. die langkettigen Zellulosen verdauen und in kürzerkettige Moleküle umwandeln können.
- Thermophile Mikroben, die oberhalb einer Aktivierungstemperatur Ablösemittel erzeugen, die z. B. die Ligninschichten von den Biomassepartikeln ablösen.

In besonders bevorzugten Aktivierungsmitteln werden mehr als drei unterschiedliche Arten von Mikroorganismen bevorzugt in Kombination verwendet.
- Anaerobe Mikroben, die Biomoleküle aufbrechen und abbauen, die also z. B. die langkettigen Zellulosen verdauen und in kürzerkettige Moleküle umwandeln können.
- Aerobe Mikroben, die mit Hilfe von Sauerstoff und oder oxidationsmitteln Stoffwechsel treiben.
- Thermophile Mikroben, die oberhalb einer Aktivierungstemperatur Ablösemittel erzeugen, die z. B. die Ligninschichten von den Biomassepartikeln ablösen.

Eine Ausführungsform des Aktivierungsmittels kombiniert die vorstehend aufgeführten Mischungen von Mikroben mit Spurenelementen.

Eine besondere Ausführungsform des Aktivierungsmittels kombiniert die vorstehend erwähnten Mischungen von Mikroben mit Gelbildnern.

Eine weitere besondere Ausführungsform des Aktivierungsmittels kombiniert die vorstehend erwähnten Mischungen von Mikroben mit Hilfsmitteln für verstärkte exotherme Reaktionen, die anschließend zur Erwärmung der Einsatzstoffmischung beitragen.

Das Verfahren wird bevorzugt bei einem TS Wert zwischen 15 und 40 durchgeführt, bevorzugt 15 bis 35 und ganz besonders bevorzugt bei 22 bis 30, da nur bei diesen Werten eine gute Wärmeübertragung auf eine Stroh-Flüssigkeit Mischung ermöglich ist, so dass die Aktivierung auch erfolgt und das gesamte Verfahren in überschaubaren Zeiträumen durchgeführt werden kann. Der TS Wert ist abhängig vom gewählten Einsatzstoff und variiert deshalb in dem oben angegebenen Bereich. Die beste Wärmeübertragung mit Stroh wird dabei im besonders bevorzugten Wertebereich erhalten, was aber nicht heißt, dass in den anderen bevorzugten Temperaturbereiche das erfindungsgemäße Verfahren nicht ebenfalls mit besonders guten Ergebnissen an erhaltener Biogasmenge durchgeführt werden kann, was jedoch von der Ligninquelle bzw. dem Einsatzstoff abhängt.

Als TS Wert (in Prozent) wird die Menge Trockensubstanz je kg Edukt bzw. Einsatzstoff bezeichnet. OTS Wert bedeutet Menge an organischer Trockensubstanz und wird vorliegend synonym zu TS Wert verwendet. Ein nasses Edukt hat einen niedrigen TS-Gehalt, eine trockenes Edukt enthält viel Trockensubstanz. Ein Edukt wird dann als nass bezeichnet, wenn der TS-Gehalt kleiner als 300 g/kg Frischmasse ist, was einem TS Wert von 30 entspricht. Nur der Vollständigkeit halber werden als trockene Edukte Edukte mit mehr als 500 g Trockensubstanz je kg Frischmasse (TS mehr als 50) bezeichnet.

Der TS Wert wird bestimmt, indem die Trockensubstanz durch Wiegen der Frischmasse und anschließender Trocknung bestimmt wird. Danach wird die getrocknete Probe erneut gewogen. Der Unterschied zwischen den beiden Messungen ist der Wasseranteil, der während des Trocknungsprozesses verdampft ist. Die Menge an Trockensubstanz wird in g/kg Frischmasse wiedergegeben. Häufig wird die Trockensubstanz auch in Prozent angegeben. Ein TS-Gehalt von 330 g/kg Frischmasse ist identisch mit 33% TS, bzw. dimensionslos als TS = 33.

Die thermische Aktivierung erfolgt durch überschreiten einer Aktivierungstemperatur Ta für mindestens den Aktivierungszeitraum Za. Dabei ist die tatsächlich erreichte Temperatur Tt ausschlagegebend dafür wie lange die Aktivierungszeit Za mindestens sein muss, um die schwer aufschließbaren Einsatzstoffanteile aufzuschließen. Biologisch bedingt ist aktuell die niedrigste Temperatur Ta = 63 °C, dann aber benötigt man noch Stunden Aktivierungszeit. Technisch notwendig sind also tatsächliche Temperaturen von Tt = 70 °C bis 75 °C, um bei angemessen kurzer Zeit die Aktivierung zu erreichen. Überraschenderweise wirde gefunden, dass die Aktivierungstemperatur nicht scharf begrenzt, sondern gleitend ist. Es herrscht eine Beziehung, wonach die doppelte Übertemperatur Tt-Ta mindestens zur Halbierung der Aktivierungszeit Za führt. Technisch wird man auch die tatsächliche Zeit Zt der Überschreitung von Ta länger machen als Za. Nebeneffekt der thermischen Aktivierung ist meist auch eine Hygienisierung des Einsatzstoffes.

Das festes Edukt, z. B. Stroh, wird vorteilhafterweise vor der fest-flüssig Mischung mechanisch zerkleinert. Die mechanische Zerkleinerung des Einsatzstoffes und die nachfolgende Mischung mit dem zugegebenen Aktivierungsmittel wird erfindungsgemäß durchgeführt. Optional wird ein Arbeitsdruck von mehr als 1 bar erzeugt, bevorzugt mehr als 2 bar oder mehr als 3 bar, um die Gasbildung zu unterdrücken und die Gaslöslichkeit zu erhöhen. Die Druckerhöhung ist nicht zwingend nötig, bei Atmosphärendruck entstehen und entweichen aber oftmals CO₂ und NH₃. Kennzeichen der Erfindung ist also im Besonderen die druckfest ausgeführte geschlossene Anlage im Bereich der Erwärmung. Der Feststoffeintrag ist natürlich offen. Hier wird wie technisch üblich z. B. Stroh über einen Trichter in einen Feststoffeintragsapparat eingeschüttet. Der Feststoffeintrag ist aber Flüssigkeitsdicht und Druckdicht abgedichtet gegen den Erwärmungsbereich oder die Erwärmungsbereiche. Die Druckentspannung erfolgt erst nach der thermischen Aktivierung, insbesondere nach Druckabbau durch Reibungsverluste im Rohr beim Verlassen des Rohrendes im Fermenter.

Das erfindungsgemäße Verfahren wird in einer weiteren Ausführungsform wie folgt durchgeführt:
Die Zubereitung beginnt mit der Bereitstellung einer Mischung aus ligninhaltiger vorwiegend organischer Trockensubstanz - Biomasse. Diese wird bevorzugt auf ca. 20 °C bis 30 °C temperiert und optional bereits zuvor mit der Aktivierungssubstanz in Kontakt gebracht, z. B. durch Berieseln etc.

Die gemischte Trockensubstanz wird in einer Feststoffzuführung komprimiert und mit einem zugeführten Fluidstrom vermischt. Dies gelingt z. B. mit dem EnergyJet der Fa. Vogelsang oder mit anderen, dem Fachmann an sich bekannten Mischern.

Der zugeführte Fluidstrom wird auf ca. 80 °C vortemperiert. Dies erfolgt durch stufenweises aufheizen bevorzugt mit Wärmerückgewinnung und in Umlaufsystemen.

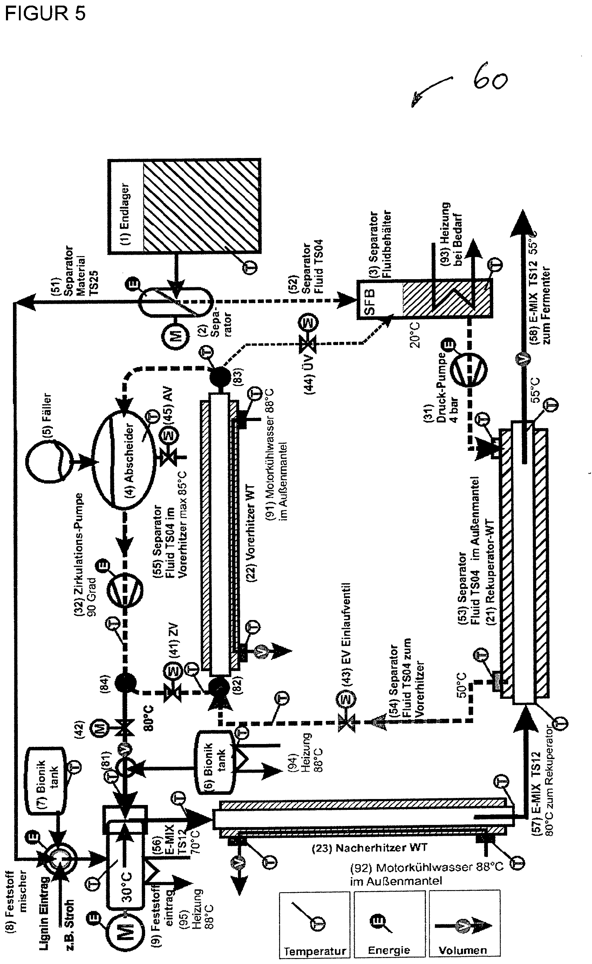

In weiteren Ausführungsformen des erfindungsgemäßen Verfahrens in der Vorrichtung mit Zirkulation kann das Verfahren getaktet durchgeführt werden (siehe Figur 5).

In Takt A wird flüssiger Einsatzstoff in den Kreislauf gefördert, wobei das Zirkulationsventil (41) geschlossen ist und das Einlaufventil (43) und das Dosierventil (42) offen sind. Zudem sind die Druckerhöhungspumpe und die Zirkulationspumpe gleichzeitig aktiv. Die vorgeheizte Einsatzstoff strömt zum Einsatzstoffmischer (9), um dort mit dem festen Einsatzstoff vermengt zu werden. Der gleiche hydrostatische Schub fördert Takt für Takt den angemischten Einsatzstoff durch den thermisch aktivierend wirkenden Wärmetauscher (23) und weiter durch den Rekuperator (21) bis zum Fermenter. Dabei können optional weitere Förderanlagen eingebaut werden, wenn der Druckabfall dies erfordert.

In Takt B ist das Einlaufventil (43) geschlossen, das Zirkulationsventil (41) ist geöffnet und das Dosierventil (42) ist geschlossen. Folglich steht die gesamte Zirkulationsleitung unter einem Arbeitsdruck und der flüssige Einsatzstoff wird durch die Zirkulationspumpe im Umlauf gefördert, bis eine gewünschte Vorheiztemperatur erreicht ist. Dann erfolgt wieder Takt A.

Das im Einsatzstoffmischer erzeugte Gemisch hat bereits teilweise die Mischtemperatur über 70 °C erreicht. Es wird weiter erhitzt auf Temperaturen bis zu 80 °C, um möglichst in allen Volumenelementen der Biomassezubereitung die Aktivierungstemperatur möglichst lange zu überschreiten. Dies kann z. B. mittels des von Motorkühlwasser beheizten Nacherhitzerwärmetauscher erfolgen, wie vorstehend beschrieben.

Optional kann anschließend eine Abkühlung der Biomasse Zubereitung erfolgen. Um eine Überhitzung der Fermenter zu vermeiden, erfolgt üblicherweise eine Abkühlung, z. B. in einem Rekuperator.

Die so aus ligninhaltiger Biomasse erzeugte Biomasse-Zusammensetzung kann anschließend in Bioraffinerien und Biogasanlagen als sehr hochwertiger Einsatzstoff verwendet werden.

Die mikrobiologische Aktivierung findet Im Nacherhitzerwärmetauscher und danach statt. Die erfindungsgemäße Aktivierung führt zunächst zur mikrobiologischen Ablösung von Lignin und anschließend zum mikrobiologischen Aufschluss von Lignin. Die Aufschlussreaktion läuft dabei auch bei wieder fallender Temperatur und auch bei wieder fallendem Druck weiter ab, bis in die Verweilzeit im nachfolgenden Fermenter. Die Aktivierung muss also nur über eine Mindestdauer von bevorzugt einigen Sekunden erfolgt sein, damit der mikrobiologische Aufschluss des Lignins nachfolgend ablaufen kann. Infolge des Aufschlusses des Lignins wird eine entsprechende Mehrmenge an nutzbaren Bioenergiestoffen, z. B. Biomethan erzeugt.

Weiterhin wird durch die erfindungsmäßige Zerkleinerung und Aktivierung der Einsatzstoffe der elektrische Energieaufwand für Rührwerke, Separatoren und andere Hilfsgeräte deutlich reduziert. Insgesamt betrachtet kann also unter günstigen Bedingungen der Aufschluss des Lignins mit sehr wenig zusätzlicher elektrischer Energie erfolgen. Dies ist wirtschaftlich und ökologisch von großem Vorteil.

Die Aufgabe der vorliegenden Erfindung wird weiter gelöst durch eine Vorrichtung zur Verwendung bei der Durchführung des erfindungsgemäßen Verfahrens zur Erzeugung einer Biomassezubereitung wie in Anspruch 13 beschrieben.

Die Vorrichtung kann weiterhin umfassen:
- eine Einrichtung zur Druckerhöhung oder Volumenförderung.

Die Einrichtung zur Druckerhöhung und / oder Volumenförderung kann beispielsweise eine Pumpe und / oder einen Kompressor umfassen.

In einer weiteren Ausführungsform umfasst eine Vorrichtung zur Erzeugung einer Biomassezubereitung:
a) eine Vorrichtung zum Einbringen von schwer aufschließbaren biologischen Edukt/en insbesondere mit hohem Ligninanteil,
b) eine Vorrichtung zur Zugabe und Untermischung eines Mittels (Aktivators / Aktivierungsmittels) zum Aufschließen des/der Edukte, wobei das Aktivierungsmittel bei Überschreiten einer Temperatur T_{A} aktivierbar ist, mit T_{A} > 55 °C, optional mit der zusätzlichen Bedingung Druck P>P_{A} mit P_{A} > 1,3 bar,
c) eine Vorrichtung zur Aktivierung einer Kombination / Mischung aus Edukt/en und Aktivator durch Temperaturerhöhung auf T>T_{A} bei Druck P>P_{A},
d) eine Vorrichtung zur Druckerhöhung- und Volumenförderung, insbesondere eine Pumpe oder ein Kompressor, bevorzugt in Flussrichtung vor der Vorrichtung zur Aktivierung angeordnet.

Die erfindungsgemäße Vorrichtung verarbeitet schwer aufschließbare, insbesondere ligninhaltige, Einsatzstoffe und ermöglicht die Erzeugung einer hochwertigen Biomassezubereitung, in der auch das Lignin zu einem großen Teil aufgeschlossen ist. Sie ist bestens geeignet für den Einsatz in Bio-Raffinerien oder Biogasanlagen.

Das Aktivator umfasst eine Mischung von mikrobiologischen Komponenten, bevorzugt eingebracht in einer Flüssigkeit und optional ergänzt durch Nährstoffe und Vitalstoffe.

Der erfindungsgemäße mikrobiologischen Aktivator / das Aktivierungsmittel enthält große Mengen (mehr als 10⁸ Teilchen / Mikroben pro Milliliter, bevorzugt 10¹⁰ bis 10¹² pro Milliliter) an thermophilen druckliebenden Mikroorganismen. Besonders bevorzugte Aktivierungsbedingungen sind Temperaturen zwischen 65 und 85 °C und Drücke zwischen 2 bar und 6 bar. Der Aktivator besteht aus einer Mischung von mikrobiologischen Komponenten, bevorzugt eingebracht in einer Flüssigkeit.

Der Aktivator ist beispielsweise eine Suspension, die mehr als 1 Mio. Teilchen je Milliliter enthält, bevorzugt mehr als 1 Mrd. Teilchen je Milliliter. Sie enthält bevorzugt sowohl aerobe, als auch anaerobe als auch thermophile Mikroorganismen. Ein Anteil dieser Mikroorganismen ist Lösemittelbildner, d. h. die Organismen erzeugen lokal auf der Oberfläche der Einsatzstoffpartikel Lösemittel, die die vorher schwer aufschließbaren Schichten angreifen und die Ligninschicht ablösen. Die Anwesenheit des Lösemittelbildners ist zwingend erforderlich. Bevorzugt ist der Lösemittelbildner der thermisch aktivierbare mikrobiologische Bestandteil. Zu unterscheiden ist die aktivierbare Lösemittelbildung von einer hier nicht stattfindenden direkten Zugabe von Lösemitteln. Die aktivierbare Bildung der Lösemittel erfolgt erfindungsgemäß mikrobiologisch, also durch Urtierchen (Archaea), Bakterien und / oder Pilze. Zu unterscheiden ist auch zwischen dem nach Aktivierung an den Grenzflächen der Biomasse gebildeten Lösemittel und den bei anderen Technologien verwendeten Lösungsmitteln. Die nach Aktivierung mikrobiologisch produzierten Lösemittel führen z. B. zunächst nur zur Ablösung der Ligninschicht, jedoch noch nicht zur Auflösung des Lignins. Erst nach der Ablösung werden die Ligninschicht-Fragmente durch Mikroben in kurzkettige Kohlenstoffverbindungen aufgelöst bzw. aufgeschlossen.

Das Edukt kann in fester, breiförmiger oder flüssiger Form eingesetzt werden, sodass nahezu alle möglichen Biomassen, wie z. B. Stroh, Pflanzenstengel, Holzspäne, tierische Exkremente, Schlachtabfälle etc. aufgeschlossen werden können.

Die erfindungsgemäße Vorrichtung kann eine Vorrichtung zum In-Kontakt-Bringen des/der Edukt(e) mit dem Aktivator beispielswiese eine Mischkammer umfassen, jedoch ist in einer weiteren Ausführungsform vorgesehen, dass das Edukt bereits vor der Einbringung in die eigentliche erfindungsgemäße Vorrichtung mit dem Aktivator in Kontakt gebracht wird, z. B. mittels Besprühen des Edukts mit einem flüssigen Aktivator.

Die erfindungsgemäße Vorrichtung zur thermischen Aktivierung umfasst eine Vorrichtung zur Temperierung der Edukte bzw. der Biomassezubereitung auf eine Temperatur oberhalb der Aktivierungstemperatur T>T_{A}. Bevorzugt werden dazu Wärmetauscher verwendet, die z. B. durch Motorabwärme eines nahe stehenden Blockheizkraftwerks beheizt werden. Die Aufheizung der mit Aktivator vermischten Edukte bewirkt den Start der Aktivierung. Bevorzugt wird die Aktivierung durch die Anwendung eines erhöhten Druckniveaus unterstützt, wobei der Druck höher ist als der Aktivierungsdruck P>P_{A}. Die Temperaturerhöhung kann in besonderen Ausführungen auch durch interne chemische Wärmequellen hervorgerufen werden, z. B. durch ablaufende exotherme Reaktionen oder durch elektrische Heizung oder durch Einstrahlung von elektromagnetischen Wellen oder Schall. Weitere Alternativen sind weiter unten anhand der Figuren näher erläutert.

Weitere Ausführungsformen der erfindungsgemäßen Vorrichtung umfassen weiterhin eine Mischvorrichtung für Edukte, die so ausgestaltet ist, dass verschiedene Einsatzstoffe mit verschiedenen Aggregatzuständen, fest, breiförmig oder flüssig, vorgemischt werden können, wie z. B. eine reine Feststoff-Vormischeinrichtung oder ein Fest-Flüssig-Einsatzstoffmischer.

Die erfindungsgemäße Vorrichtung weist in einigen Ausführungsformen eine Vorrichtung zur Wärmerückgewinnung auf, sodass die erfindungsgemäße Vorrichtung besonders energieeffizient betrieben werden kann. Dabei wird bevorzugt thermische Energie aus dem aufgeschlossenen Produkt auf mindestens einen der Einsatzstoffe übertragen, um dessen Temperatur zu erhöhen.

Die verschiedenen Ausführungsformen der erfindungsgemäßen Vorrichtung lassen sich als eine Kombination aus einer biologischen, mechanisch und / oder thermischen wirkenden Vorrichtung beschreiben:
- Eine biologische wirkende Vorrichtung zur Zudosierung von mikrobiologischen Aktivierungssubstanzen (des Aktivators), die ab einer bestimmten Mindesttemperatur ligninhaltige Schichten angreifen können,
- eine mechanisch wirkende Vorrichtung für die Zuführung von schwer aufschließbaren Einsatzstoffen (Edukten) und Vermischung mit dem Aktivator, wobei ein bestimmtes Druckniveau erreicht wird,
- sowie eine thermisch wirkende Vorrichtung zur Einstellung einer Temperatur oberhalb der Aktivierungstemperatur, wodurch die Aktivierung des Aktivators erreicht und der mikrobiologische Aufschluss des Lignins gestartet wird.

In einer weiteren Ausführungsform kann die erfindungsgemäße Vorrichtung folgende Komponenten umfassen:
- eine Vorrichtung zur Zubereitung einer Biomassemischung, z. B. fest-flüssig Eintragssystem, Vorfermenter, Hydrolysestufe oder Desintegrationsstufe, wobei am Ausgang ein Massestrom von ligninhaltiger Biomasse zur Verfügung gestellt wird,
- eine Vorrichtung zur dosierten Zufuhr eines Aktivierungsmittels, das erst durch Erreichen bzw. Vorliegend eines aktivierenden Bereichs in Druck, Temperatur und Nährstoffkonzentration sowie Zeit aktiviert wird.
- eine Vorrichtung zur Einstellung eines Druckes z. B. 4 bar und zur Förderung, z. B. Pumpe oder Kompressor,
- eine Vorrichtung zur Einstellung einer Medientemperatur, z. B. Wärmetauscher oder Heizung oder interne biochemische Wärmefreisetzung.

Die erfindungsgemäße Vorrichtung kann in einer Weiterbildung zwei Erhitzersysteme umfassen, nämlich einen Vorerhitzer und einen Nacherhitzer. Dabei erwärmt der Vorerhitzer flüssigen Einsatzstoff auf eine Vormischtemperatur T_{V}. Der vorerhitzte flüssige Einsatzstoff wird gemischt mit einer festen Einsatzstoffzubereitung, die bevorzugt kleinstückig ist, z. B. gehäckseltes oder gemahlenes Stroh. Die entstehende Mischtemperatur ist T_{M}. Der gemischte Einsatzstoff wird vor oder nach der Mischung mit Aktivierungsmittel versetzt und anschließend durch einen Nacherhitzer auf T>T_{A} erwärmt. Dadurch kommt es zur Aktivierung des Aktivators und folglich zur mikrobiologischen Ablösung des Lignins und im weiteren zum mikrobiologischen Aufschluss des Lignins.

Die erfindungsgemäße Vorrichtung kann in einer Weiterbildung zwei Pumpensysteme, die der Druckerhöhung und Volumenförderung dienen, umfassen, nämlich ein erstes System zum Aufbau eines Arbeitsdruckes und ein zweites Pumpensystem zur Erzeugung einer Zirkulationsströmung. Die Zirkulationsströmung wird bevorzugt benutzt, um den Vorerhitzer mit flüssigem Einsatzstoff zu durchströmen.

Die erfindungsgemäße Vorrichtung kann in einer Weiterbildung drei Wärmetauschersysteme, mindestens zwei Pumpen, einem Separator zur Bereitstellung von Separatorfluid sowie einem Eintragssystem für feste Biomasse unter Zumischung von fluider oder flüssiger Biomasse umfassen.

Die drei Wärmetauschersysteme sind vorzugsweise so ausgelegt, dass sie je bis zu 50 Kilowatt Wärmeleistung übertragen können, bezogen auf eine Feuerungswärmeleistung von 500 Kilowatt. Der erste Wärmetauscher fungiert als Vorerhitzer, der die vom Rekuperator auf ca. 50 °C vorgewärmte Separatorfluid auf bis zu 80 °C im Zirkulationsbetrieb erhitzt. Das Fluid durchströmt eine Zirkulationspumpe auf hohem Druckniveau von ca. 4 bis 10 bar. Der zweite Wärmetauscher fungiert als Nacherhitzer, der Mischung aus Biomasse / Aktivator nochmals auf Temperaturen von mehr als 75 °C, bevorzugt 80 °C erhitzt, um den Ligninaufschluss zu starten. Der dritte Wärmetauscher fungiert als Rekuperator zur Rückgewinnung von Wärme und überträgt z. B. im Gegenstrom die Wärme von der erfindungsgemäß aufgeschlossenen Biomassezubereitung auf das bereit gestellte Separatorfluid, den flüssigen Einsatzstoffanteil. Das ist wichtig, um den Wärmeeintrag in den Fermenter zu begrenzen. Die Wärmetauscher sind bevorzugt als Rohrmantelwärmetauscher ausgestaltet. Im durchgehenden Innenrohr fließt jeweils das höher viskose Fluid. Das Außenrohr ist bevorzugt gewendelt und hat zwei Flansche für Zu- und Ablauf. Der gesamte Wärmeaufwand liegt bei etwa 6 % bis 20 % der Feuerungswärmeleistung. Die Wärme kann z. B. über das Motorkühlwasser eines BHKW regenerativ bereit gestellt werden.

Die Druckerzeugung und Förderung von Einsatzstoffen mit hohem Trockensubstanzgehalt ist technologisch schwierig, insbesondere bei hoher Temperatur über 60 °C. Folglich arbeitet eine besonders bevorzugte Ausführungsform der erfindungsgemäßen Vorrichtung mit einer Druckerzeugungspumpe, die kühlen fluiden Einsatzstoff mit T<50 °C komprimiert auf einen Vordruck Pv von z. B. 4 bar bis 6 bar. Dann erst erfolgt die Erwärmung des fluiden Einsatzstoffes. Durch den erhöhten Arbeitsdruck wird die Gasfreisetzung unterbunden. Der Arbeitsdruck wird im Weiteren als vorwiegende Antriebskraft benutzt, um die Masse durch Rohre, Mischer, Ventile und Wärmetauscher etc. zu bewegen.

Die Vorwärmung des Einsatzstoff-Fluids wird ergänzt durch einen weiteren Wärmetauscher, der die Möglichkeit hat, auch ohne Einmischungsförderung von Fluid in den Einsatzstoffmischer das Fluid zur Wärmeaufnahme zu bringen. Besonders bevorzugt ist ein Umwälz-Heizkreislauf, angetrieben von einer zusätzlichen Umwälzpumpe, der sich auf Kreislaufdruck Pk befindet. Die Pumpe fördert von Druck Pkr auf Pkv. Der Druckabfall im Heizkreislauf erfolgt über Rohre, Verteiler, Ventile Wärmetauscher etc.

Der Zugang zum Fluideintrag in den Einsatzstoffmischer ist durch mindestens ein Ventil Ve abgeschlossen, das in seiner Öffnung variiert werden kann. Der Umwälzkreislauf hat ebenfalls ein Ventil Vk, das den Kreislauf frei gibt oder absperrt. Die erste Position ist die Befüllung des Kreislaufs mit geschlossenem Kreislauf-Ventil Vk und geöffnetem Eintragsventil Ve. Die zweite Position ist die Umwälzposition mit geöffnetem Kreislauf-Ventil Vk und geschlossenem Eintragsventil Ve. Mischpositionen dazwischen sind möglich und manchmal vorteilhaft.

Eine Weiterbildung der Erfindung umfasst weiter einen Einsatzstoffmischer für feste Eintragsstoffe, optional versehen mit Aktivator, und fluide Eintragsstoffe, bevorzugt vorgeheizt. Der Einsatzstoffmischer umfasst eine Verdichtungsstrecke mit Motorantrieb, die einen flüssigkeitsdichten Stopfen aus Einsatzstoff bildet und laufend nachbildet, in dem Maße wie im Einsatzstoffmischer fester Einsatzstoff zugemischt wird. Der kontinuierlich gebildete und verbrauchte Stopfen bewirkt eine druckfeste Abdichtung gegen den unter Druck zugeführten flüssigen Einsatzstoff und gegen den Aktivierungsbereiche, in dem der gemischte Einsatzstoff thermisch aktiviert wird.

In einer speziellen Weiterbildung wird die Dichtheit des Stopfens durch eine neuartige Regeltechnik sichergestellt. Die bisher übliche Regeltechnik regelt die Drehzahl der Druckerzeugungspumpe. Überraschend wurde festgestellt, dass dies nicht die gewünschte Sicherheit gegen Durchschlag der Flüssigkeit gegen die Förderrichtung liefert. Erfindungsgemäß wird nun ein Regelventil im Einsatzstofffluidzulauf geregelt und optional zur Sicherheit eine Absperrschieber in Serie geschaltet, womit der Durchfluss schnell reduziert wird, falls die Dichtheit am Einsatzstoffmischer nicht mehr vorhanden ist. Der Einsatzstoffmischer erzeugt selbsttätig durch seine Aktion einen fortlaufend verbrauchten Einsatzstoffstopfen, der abdichtende Eigenschaften besitzt. Diese Abdichtungseigenschaften sind umso besser, je besser sich die Masse kompaktieren lässt, so dass keine Fluidkanäle verbleiben.

In einer Weiterbildung umfasst die erfindungsgemäße Vorrichtung eine Zuführung von gelbildenden Mitteln zum trockenen Einsatzstoff. Denn in einer Testsituation mit schlecht abdichtenden Einsatzstoffen wurde überraschend festgestellt, dass eine Zugabe von gelbildenden Mitteln in den Vormischer in die Mischung der festen Einsatzstoffe oder aber in den Einsatzstoffmischer selbst zur Wirkung hat, dass sich besonders druckdichte Stopfen bilden. Diese Ausführungsform umfasst also einen Einsatzstoffmischer mit Zuführungsvorrichtung für Gelbildner. Geeignete Gelbildner sind z. B. Chitin- oder Chitosan-Zubereitungen oder Öle oder geleeartige Zubereitungen, insbesondere solche, die bei erhöhten Temperaturen flüssig werden und bei Temperaturen unter 60 °C geleeartig sind. Die zugegebenen Mengen führen dazu, dass die Fluidkanäle im Stopfen verschlossen werden. Der Stopfen indes wird in Betrieb stets verbraucht und das Material wird zugemischt. Man muss also ständig neue Gelbildner zumischen.

### Figuren und Ausführungsbeispiele

Der Erfindung ist nachstehend anhand von Ausführungsbeispielen und Figuren näher erläutert ohne dass dies als auf eine bestimmte Ausführungsform als beschränkend verstanden werden soll.

Es zeigen:
- Figur 1:: ein Schema der erfindungsgemäßen Vorrichtung,
- Figur 2:: eine Ausführungsform der erfindungsgemäßen Vorrichtung mit einem Einsatzstoffmischer für feste Einsatzstoffe,
- Figur 3:: eine Ausführungsform der erfindungsgemäßen Vorrichtung mit einem Einsatzstoffmischer für feste und flüssige Einsatzstoffe,
- Figur 4:: eine Ausführungsform der erfindungsgemäßen Vorrichtung mit einer Vorrichtung zur Wärmerückgewinnung, und
- Figur 5:: eine weitere Ausführungsform der erfindungsgemäßen Vorrichtung in schematischer Ansicht.
- Figur 6:: einen Vergleich der Menge an erhaltenem Biogas beim erfindungsgemäßen Verfahren und beim Verfahren des Standes der Technik.

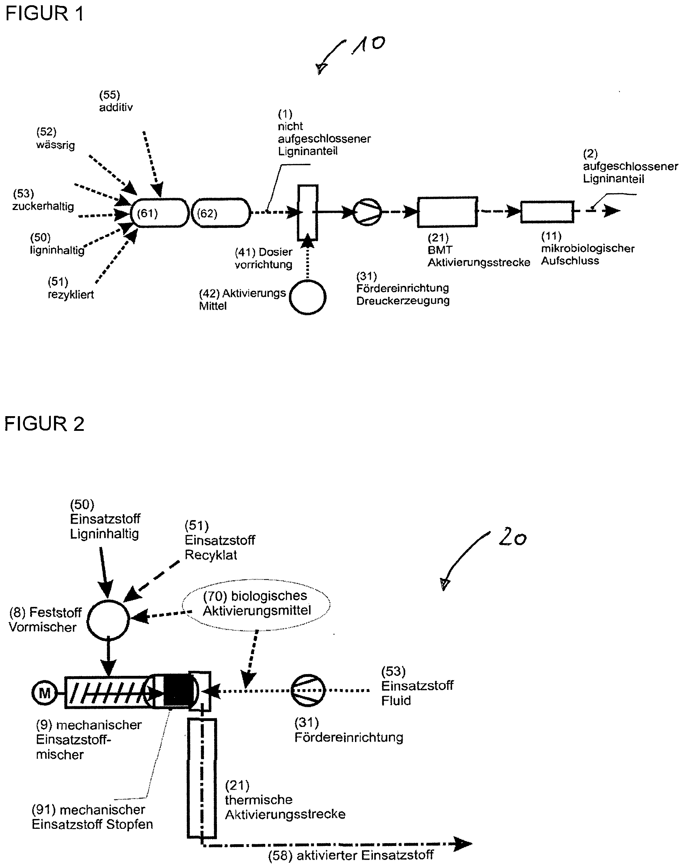

In Figur 1 werden verschiedene Einsatzstoffe (50, 51... 55), die in einer Vorbereitungsvorrichtung 61-62 einsatzfähig gemacht. Diese Vorbereitungsvorrichtung 61-62 können beispielsweise Fest-Flüssig Einsatzstoffmischern wie EnergyJet, flüssig-flüssig-Einsatzstoffmischer, Vorfermenter, Hydrolysestufen oder thermische Desintegratoren oder Separationsvorrichtungen sein. Allgemein wird so der erfindungsgemäßen Vorrichtung ein pumpbares fließfähiges und wärmeleitendes Biomasse-Material (1) zur Verfügung gestellt, das einen wirtschaftlich interessanten Gehalt an schwer aufschließbarer ligninhaltiger Biomasse aufweist (bei Stroh als Einsatzstoff sind bei herkömmlichen Systemen bis zu 60% und mehr nicht in den für wirtschaftliche Anlagen relevanten Verweilzeiten aufschließbar). Typisch für das Biomassematerial (1) sind 30 % bis 80 % der organischen Substanz als ligninhaltige Biomasse. Um den bisher nicht möglichen Aufschluss von Lignin und damit die weitere Freisetzung von Energieträgern wie CH₄ (Methan) zu ermöglichen wird ein Aktivator zugegeben und anschließend aktiviert. Die Zugabe kann im Grunde bereits in der Vorbereitungsstufe (61-62) erfolgen, sie muss spätestens vor der Aktivierungsstufe (21) erfolgen. Die gesamte Aktivierungsvorrichtung besteht aus mehreren Elementen (41, 31,21 etc.) und stellt die geeigneten Aktivierungsbedingungen her. Die Zugabe von Aktivator (42) erzeugt dabei die biologischen Voraussetzungen her (durch Zugabe von thermophilen aktivierend wirkenden Mikroorganismen, die lokal an der Lignin-Biomasse ablösend wirkende Biochemikalien erzeugen können, ergänzt bei Bedarf durch Hilfsstoffe wie Spurenelemente). Die Zugabe des Aktivators erfolgt mittels Dosiersystem in Mengen von typisch 0,1 bis 10 Liter pro Tonne gesamte Biomasse. Das Dosiersystem (41) ist optional so ausgelegt, dass es auch auf das erhöhte Aktivierungsdruckniveau (z. B. 4 bar) eindosieren kann. Nach der Eindosierung wird Biomasse und Aktivator vermischt und kontaktiert, z. B. durch die Mischwirkung eines fest-flüssig Eintragssystems (z. B. EnergyJet) oder die Mischwirkung einer Fluid-Pumpe (z. B. Kreiselpumpe). Zusätzlich zur Mischung wird in diesem Förderelement (31) auch eine Druckerhöhung auf Aktivierungsdruck größer Pa vorgenommen. Zudem wird eine Temperaturerhöhung auf Aktivierungstemperatur größer Ta vorgenommen. Für die Temperaturerhöhung wird ein Erhitzungselement (21) eingesetzt ausgewählt aus (Wärmetauscher, Heizpatrone, interne reaktive Wärmequelle, z. B. exotherm reagierende Zusätze). Nach der Aktivierung (spätestens einsetzend in Aktivierungsvorrichtung 21) erfolgt zunächst die Ablösung der Ligninhaltigen Biomasse und danach schrittweise die Auflösung der ligninhaltigen Biomasse, wobei die sonst schwer aufschließbare Biomasse in eine vergärbare Substanzmischung umgewandelt wird, die insbesondere kurzkettige Kohlenstoffverbindungen enthält.

In Figur 2 zeigt eine erfindungsgemäße Vorrichtung mit Einsatzstoffmischer, hier bezeichnet als (9). Der Mischer (9) erzeugt den Einsatzstoff (1) für das eigentliche Aktivierungs-System. Man sieht auch, dass die Position der Pumpe (31) in den Fluidkreis gesetzt ist. Entscheidend ist der Druck nach dem Mischer (9) und in der thermisch Aktivierungsvorrichtung (21) für den gelten muss P>Pa sowie T>Ta. Der danach aktivierte Einsatzstoff ist hier mit (58) bezeichnet.

Dem Einsatzstoffstrom wird der Aktivator (42) zugegeben, wahlweise in die EinsatzstoffMischung (50,... , 55) oder in den daraus erzeugten Einsatzstoff-Mix (1). Zudem wird der Einsatzstoff (1) auf Druck P>Pa und auf Temperatur T>Ta gebracht. Dadurch wird die Aktivierung erreicht und die mikrobiologischen Komponenten des Aktivators (42) beginnen aktiv zu werden. Nach der Aktivierung erfolgt eine Reaktionsstrecke, wobei hier die Temperatur bereits wieder abgesenkt werden kann, aber nicht muss. Temperaturabsenkung durch z. B. Rekuperator wird optional benutzt, im bei mesophilen Fermentern eine Temperaturerhöhung im nachfolgenden Fermenter zu begrenzen. Bei Thermophilen Fermentern kann man z. B. auf die Abkühlung nach Aktivierung verzichten. Eine besonders bevorzugte Ausführung benutzt zur Erzeugung des Einsatzstoff-Mix (1) einen Anteil von festen Gärresten, bevorzugt aus dem Endlager. Diese werden z. B. durch eine Separationsvorrichtung (Filter, Separator, etc.) abgetrennt und zum Einsatzstoff-Mischer gebracht und dort mit Stroh vermengt. Eine weitere besonders bevorzugte Ausführungsform nutzt einen Anteil flüssiger Gärreste oder Fermenterinhalte mit org. Trockensubstanzgehalt typisch unter 10 %, bevorzugt unter 6 %, besonders unter 4 %, um diesen fluiden Anteil separat aufzuwärmen, zu komprimieren und mit Aktivator zu versehen. Anschließend erfolgt die Vermischung mit den trocken-festen Einsatzstoffen in einer dafür geeigneten Mischvorrichtung wie z. B. EnergyJet von Vogelsang. Die so entstehende Einsatzstoff Mischung ist durch die Zugabe des Aktivators und durch die Mischung bereits aktivierungsfähig. Auch der Vordruck kann genutzt werden, um den Aktivierungsdruck zumindest teilweise aufzubauen. Auch die nach Mischung erhaltene Vorlauftemperatur Tv ist eine gute Ausgangsposition für die weitere Erwärmung über die Aktivierungstemperatur Ta hinaus. Im Sonderfall reicht die Mischer-Vorlauftemperatur Tv sogar aus, um die Aktivierungstemperatur Ta zu erreichen. Im Regelfall aber wird eine weitere Erwärmungsstrecke (21) nachgeschaltet, um Ta zu überschreiten. Erwärmung entweder durch Wärmetausch z. B. mit Motorkühlwasser oder durch Elektroheizer mit Überschussstrom oder durch enthaltene exotherme Reagenzien.

Besonders bevorzugt ist weiterhin die Führung des Fluids mit geringem Gehalt an Organische Trockensubstanz (OTS) in einer Vorerhitzungsvorrichtung, z. B. Wärmetauscherkreislauf, auf einem erhöhten Druckniveau Pv, das oberhalb des Aktivierungsdruckes Pa liegt. Dazu wird eine Druckerhöhungsstufe (31) verwendet und optional eine weitere Umwälzvorrichtung (32). Die Zugabe des Aktivators (42) erfolgt wahlweise vor dem Einsatzstoffmischer (fluidseitig oder feststoffseitig, nutzt die Mischwirkung aus) oder nach dem Einsatzstoffmischer (dann aber kombiniert mit nachfolgendem Aktivator-Mischer als Teil von (41)), in jedem Fall aber vor der thermisch wirkenden Aktivierungsvorrichtung (21). Den Aktivator (42 bzw. 70) kann auch nach dem Mischer (9) zugegeben werden, was aber nicht vorteilhaft ist, weil dann ein zusätzlicher Aktivator-Mischer (41) nötig wäre. Bevorzugt ist die Zugabe des Aktivators (42) vor dem Einsatzstoff-Mischer (9) bzw. in das System (61-62) in Figur 2, weil dann die Mischwirkung des Einsatzstoffmischers für die innige Vermengung mit dem Aktivator (42 bzw. 70) genutzt wird.

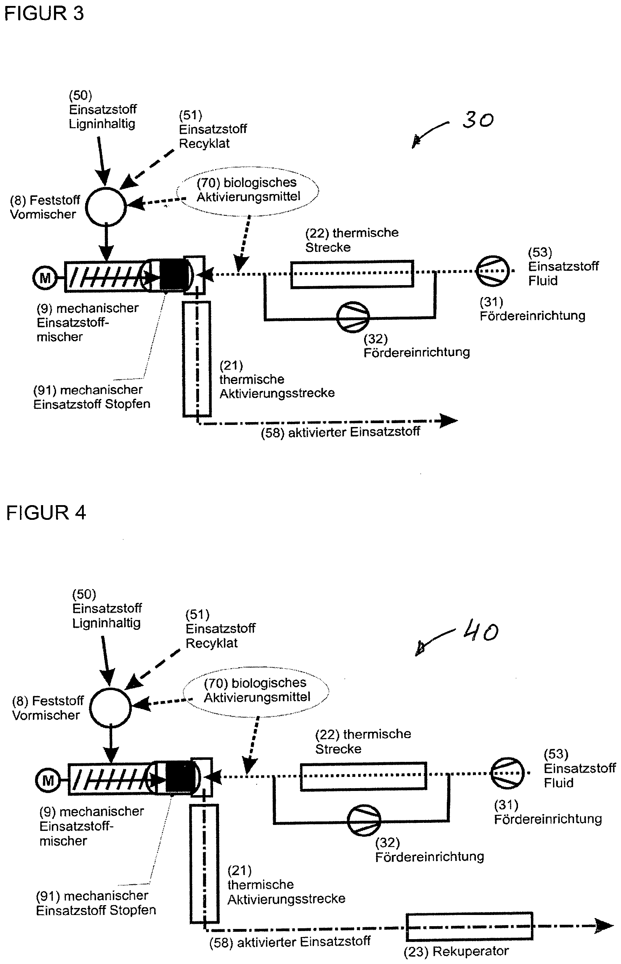

Figur 3 zeigt eine weitere Ausführungsform der erfindungsgemäßen Vorrichtung mit einer Vorrichtung für die Mischung von festen und flüssigen Einsatzstoffen. Nach dem Mischer 9) ist der Einsatzstoff (1) für die Aktivierung verfügbar. Die Vorheizung der fluiden Einsatzstoffe (53) befolgt über eine thermische Strecke (22), z. B. einen Wärmetauscher oder ein Heizelement, optional erweitert durch Umlaufkreis mit Fördereinrichtung 32. Der Aktivierungsdruck wird bevorzugt aufgebaut durch eine Druckerhöhungsstufe bzw. Fördereinrichtung (31). Die Erwärmungsstrecke (21) stellt sicher, dass im Einsatzstoff überall mindestens T>Ta erreicht wird. Der Aktivierte Einsatzstoff (58 bzw. 2) kommt in die Reaktionsstrecke, in der das abgelöste Lignin mikrobiologisch aufgelöst bzw. aufgeschlossen wird. Optional wird dabei oder danach die Temperatur des aktivierten Einsatzstoffes reduziert. Figur 3 zeigt weitere besondere Pumpenanordnung. Dabei erzeugt die Fördereinrichtung (31) bereits den hohen Arbeitsdruck. Fördereinrichtung 32 dient der Zirkulation des fluiden Einsatzstoffes (53) im Vorheizkreis inkl. Thermischer Strecke (22) die insbesondere als Wärmetauscher ausgeführt werden kann, beheizt z. B. durch Motorabwärme.

Anstelle eines Wärmetauschers als Ausführungsform für die Erwärmungsstrecke (21 und oder 22) könne ebenso elektrische Erhitzer oder biochemische Reaktionskomponenten, die zur Erwärmung führen verwendet werden. Wesentlich ist lediglich, dass in diesen Erwärmungsstrecken ein thermischer Zustand T>Ta erreicht und über Zeit Z>Za gehalten wird.

Auf die Verwendung eines Wärmetauschers kann verzichtet werden, wenn nach der Zumischung des Aktivators die Einsatzstoffmischung auf eine förderfähige Konsistenz gebracht wird, z. B. durch Mischen mit Fluiden, und davor und oder danach infolge von besonderer Wärmefreisetzung auf eine tatsächliche Temperatur Tt oberhalb der Aktivierungstemperatur Ta gebracht und mindestens die Zeit Za auf mindestens dieser Temperatur gehalten wird. Die besondere Wärmefreisetzung kann z. B. durch exotherme Reaktionen erfolgen, wie sie z. B. unter Nutzung von eingebrachtem Oxidationsmitteln und oder anderen Reaktionsmitteln erfolgen können. Die Einbringung von Sauerstoff z. B. geschieht bei der Feststoffzufuhr aus dem atmosphärischen Raum und es können zusätzliche Reaktionsfähige Mittel zugegeben werden.

In Figur 4 ist eine weitere Variante der in Figur 3 näher beschriebenen Vorrichtung gezeigt. Diese weist nun zusätzlich eine Vorrichtung zur Wärmerückgewinnung auf (Rekuperator). Die Einsatzstoffmischung hat nach der Aktivierung zunächst eine hohe Temperatur größer Ta und transportiert somit viel spezifische Wärme. Diese Wärme kann z. B. abgegeben werden, um das Einsatzstofffluid vorzuwärmen. Es handelt sich also um eine Wärmerückgewinnung, die den Wärmeverbrauch der Anlage reduziert. Zudem reduziert sich der Wärmeeintrag in den Fermenter. Bevorzugte Temperaturen nach dem Rekuperator sind z. B. 55 °C. Verglichen mit der in Figur 3 gezeigten Ausführungsform weist die Vorrichtung gemäß Figur 4 also einen Rekuperator (23) zur Wiedergewinnung der Wärme aus dem aktivierten Einsatzstoff (58) auf. Die wieder gewonnene Wärme kann z. B. zur Vorwärmung des fluiden Einsatzstoffes (53) verwendet werden. In einer weiteren Ausführungsform kann der Rekuperator (53) auch einen einfachen Kühler beheizen.

In Figur 5 ist ein schematischer Aufbau einer erfindungsgemäßen Vorrichtung gezeigt. In dieser speziellen Ausführungsform wird insbesondere das Aufschwimmen von gehäckseltem Stroh durch Aktivierung und Vermischung von ligninhaltiger Biomasse mit Aktivierungsfluid und Rezyclat in der Vorrichtung für Feststoffeintrag (9) vorteilhafterweise vermieden.

Die Schwierigkeit der Wärmeübertragung auf eine Stroh-Wasser Mischung wird dadurch gelöst, dass zunächst ein dünnflüssiges Fluid mittels Separator (2) absepariert wird, das sich gut pumpen lässt. Dieses wird im Rekuperator (21) vorgewärmt und im Vorerhitzer-Kreislauf per Vorerhitzer-Wärmetauscher (22) auf ca. 70 °C bis 80 °C erwärmt wird. Als Wärmequelle dient z. B. Motorkühlwasser.

Im Umlaufverfahren oder periodisch wird das Ventil (41) geschlossen und über das Dosierventil (42) aus dem Vorheizkreis die nötige Menge Rezyklat / Separatorfluid entnommen und mit Überdruck von z. B. bis zu 6 bar in die vorbereitete organische Masse gepresst.

Die ligninhaltige Biomasse muss erfindungsgemäß mindestens einige Sekunden auf eine Temperatur von bevorzugt 75 °C, besonders bevorzugt 80 °C erwärmt werden.

Direkt nach dem Feststoffeintrag hat die Biomasse-Aktivator-Mischung eine Mischtemperatur unter 70 °C. Danach wird in Wärmetauscher nacherhitzt auf ca. 80 °C. Dadurch wird die mikrobiologische Reaktion angestoßen und die Ligninaufspaltung beginnt.

Nach der Aufspaltung wird Mischung optional abgekühlt, z. B. im Gegenstrom-Wärmetausch. Die Temperatur sinkt dabei unter 60 °C und wird dem Fermenter zugeführt. Im Fermenter wird die Biomassezusammensetzung der Fermentation zugeführt. Nun sind die zuvor ligningeschützten Biomasseanteile aufgeschlossen und werden zu Biogas umgesetzt, das dann in einem Blockheizkraftwerk umgesetzt werden kann und das dann für das erfindungsgemäße Verfahren die nötige Energie liefert, so dass ein geschlossener Energiekreislauf entsteht.

Dies bedeutet je nach Einsatzstoff (z. B. Stroh) Mehrerträge von bis zu 300 % (bis zu 600 Kubikmeter je Tonne org. Trockensubstanz) verglichen mit den Verfahren des Standes der Technik erhalten werden.

### Ausführungsbeispiele

(Die Bezugszeichen beziehen sich auf Figur 5)

### Beispiel 1

Bei Repowering von Bestandsanlagen hat das Endlager anfangs noch hohe Feststoffanteile. Aus dem Endlager (1) wird Gärrest entnommen und zu einem Separator (2) geführt. Dieser trennt den Gärrest in einen Festanteil (51) mit z. B. 25 % OTS und einem Fluidanteil (52) von z. B. 4 % OTS. Das Separator Fluid wird zwischengelagert im Separator Fluidbehälter (3) und bei Bedarf frostschützend geheizt.

Der Festanteil (51) wird ebenfalls zwischengelagert und bei Bedarf zur Eintragsseite zum Feststoffmischer (8) gefördert, um dem Eintragsstoff zugemischt zu werden. Der Fluidanteil (52) mit z. B. 4 % OTS wird aus dem Lagerbehälter (3) von einer Druckerzeugenden Pumpe (31) angesaugt und auf einen Vorlaufdruck von bis zu 5 bar komprimiert.

Mit etwas Druckabfall durchströmt dieses niederviskose und chemisch alkalische Separator Fluid (53) durch einen Wärmetauscher (21), in dem es Wärme von einem erhitzten Gegenstrom (57) aufnimmt. Der Gegenstrom ist das fertige erhitzte und aktivierte Einsatzstoffgemisch auf dem Weg zum Fermenter (58).

Das vorgewärmte Separator Fluid wird in eine Zulauf Mischkammer (82) gepresst und erzeugt auf diese Weise im Zirkulationsumlauf (55) einen Systemdruck von bis zu 4 bar. der Zulauf ist hier nur bei geöffnetem Ventil (43) möglich, Optional baut man im Zirkulationskreis (55) des Vor-Erhitzers einen Pufferspeicher mit Abscheider (4) ein, der ein auffüllbares Speicher-Element enthält.

Der Zirkulationskreis (55) wird angetrieben durch eine Zirkulationspumpe (32), die weniger auf Druckaufbau ausgerichtet ist, dafür aber besonders korrosionsfest und besonders temperaturfest ausgelegt ist. Die zu erzeugende Druckdifferenz ist die des Druckverlustes im Zirkulationskreis durch die Wärmetauscher (22) und die (82, 83, 84) Mischkammern.

Der Vor-Erhitzer (8) ist als Rohrwärmetauscher ausgeführt. Erhitzt wird im Gegenstrom durch Motorkühlwasser, das dem Biogas BHKW (Blockheizkraftwerk) entnommen wird. Die Erhitzung erfolgt also 100 % regenerativ.

Der Zirkulationskreis durchströmt eine Auslass Mischkammer (84), an der die Schnelle reduziert ist, so dass ein Staudruck auf das Dosierventil (42) wirkt. Wenn das Motorventil (42) öffnet, kann erhitztes Separator Fluid (55) mit ca. 80 °C oder höher zum Feststoffeintrag (9) strömen.

Auf dem Weg zum Feststoffeintrag (9) befindet sich eine Einspritzung für den Aktivator (Bionik Fluid) aus Tank (6) und gereinigtem Heißwasser, diese Mündet in Kammer (81).

Der Einsatzstoff Eintrag erfolgt über einen Feststoffmischer (8). Dieser mischt frische Biomasse nach Bedarf mit Rezyclat (51) aus dem Separator (2) und zerkleinertem Stroh und fügt zudem eine funktionell sehr wichtige biologische Komponente aus dem Tank (7) hinzu, den Aktivator. Der Einsatzstoff wird hierbei auch zerkleinert auf eine Korngröße / Stiftlänge unter 30 mm, bevorzugt ca. 10 mm.

Der vorgemischte Einsatzstoff wird in der Feststoffeintragsvorrichtung (9) komprimiert und mit dem vorgeheizten Separator Fluid aus Kammer (81) innig gemischt. Dabei ist bevorzugt das Separator Fluid der größere Massenanteil und hat zudem die höhere spezifische Wärme. Es entsteht die Biomassezubereitung (56) mit einer Mischtemperatur z. B. unter ca. 70 °C. Diese Biomassezubereitung (56) ist mit Aktivator versetzt aber noch nicht ganz aktiviert. Sie wird also weiter erhitzt bis zur Aktivierung und dann im Rekuperator etwas abgekühlt. Von dort aus geht es zum Fermenter, wo Biogas entsteht, das abgeleitet bzw. aufgefangen und in einem BHKW eingesetzt wird, dass wiederum dem vorbeschriebenen Verfahren für die Aktivierung bzw. danach in den PreMix Stufen über zB Heißwasserleitungen die nötige thermische Energie zuführt, die durch die Verbrennung des Biogases erhalten wird.

### Beispiel 2

Im Dauerbetrieb wird die Biomasse Zubereitung aus dem erfindungsgemäßen System im Fermenter fast vollständig verwertet. Dadurch sinkt der organische Feststoffgehalt (TS bzw. O (= organisch) TS) nach einigen bis etlichen Monaten im Endlager (1) auf Werte unter 4 %. Nun ist kein Separator (2) mehr erforderlich. Die Endlagerflüssigkeit kann direkt als flüssiger Einsatzstoff (52) verwendet werden.

### Beispiel 3

Als Einsatzstoff soll z. B. vorwiegend trockenes Stroh verwendet werden, z. B. weil dieses in einem landwirtschaftlichen Großbetrieb als Abfall anfällt. In diesem Fall muss das Stroh gehäckselt werden. Der TS Gehalt von Stroh liegt über 85 %. Zur Verarbeitung muss eine Zumischung von Flüssigkeit erfolgen, z. B. Fermenterflüssigkeit. Diese hat aber zunehmend hohen Mineralgehalt. Folglich wird zudem Wasser in die Einsatzstoffzubereitung zugemischt werden, um die Mineralstoffkonzentration in Fermenter und Endlager auf Dauer nicht zu hoch werden zu lassen.

### Beispiel 4

Bei Hühnermist als Einsatzstoff gibt es ein Überangebot an Ammonium. Dann wird aus einem Dosierbehälter (5) in den Abscheider (4) ein Fällmittel eindosiert, um Ammoniumdünger aus der erhitzten zirkulierenden Flüssigkeit (55) auszufällen. Auf diese Weise können auch Metallionen und andere Bestandteile aus dem Vorerhitzerkreislauf ausgefällt werden.

### Beispiel 5

Der Biomasse Fluidkreislauf soll gespült werden. Hierfür wird das Einlaufventil (43) geöffnet und das Zirkulationsventil (41) geschlossen. Das Dosierventil (42) wird geöffnet und der Feststoffeintrag wird bei Bedarf bzw. Undichtheit überbrückt (nicht gezeichnet). Dann strömt die von den Pumpen (31) und (32) beförderte Fluid durch alle Wärmetauscher und reinigt diese. Nach dem Rekuperator (21) kann das Fluid auch wieder in den Behälter (3) gepumpt werden (nicht gezeichnet).

### Beispiel 6

Biomassen mit extrem hohen Ligningehalten von 20% und mehr bezogen auf die Gesamttrockenmasse erfordern eine besonders starke Aktivierung bei erhöhten Temperaturen. Dann wird zunächst das Dosierventil (42) geschlossen und das Separatorfluid (55) zirkuliert durch den Wärmetauscher(22) und erwärmt sich auf bis zu 85 %. Entsprechend wird nur wenig Feststoff zugeführt und es ergibt sich eine erhöhte Mischtemperatur im E-Mix (56). Diese wird weiter gesteigert im Nacherhitzer (23). Bei Bedarf könnte auch der Rekuperator (21) auf Heizbetrieb umgestellt werden, um die Aktivierung des Fluids (57) noch weiter zu steigern.

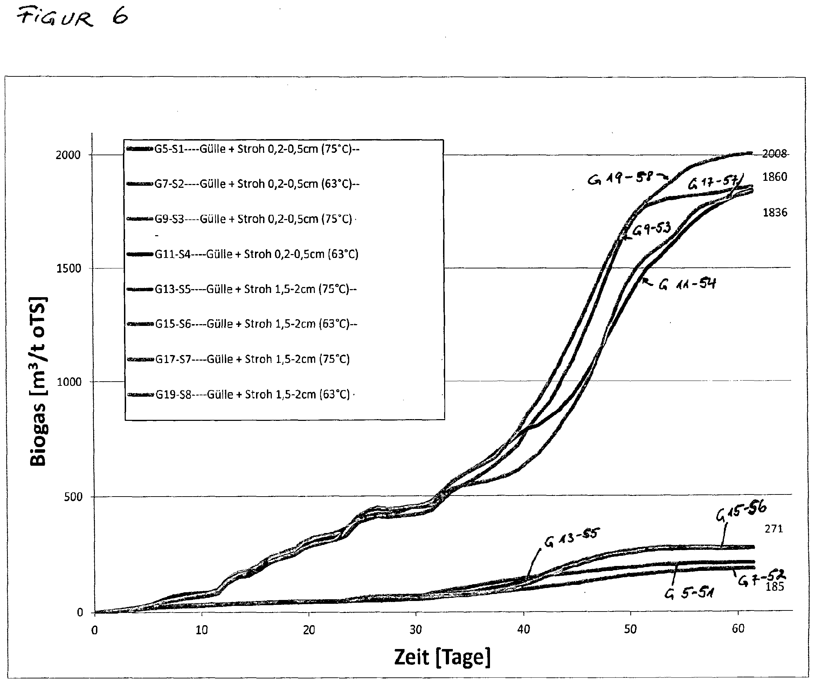

Figur 6 zeigt die Ergebnisse der Produktion von Biogas (60% Methan, 37% CO₂ und 3 % Wasser) aus einer Mischung aus Dünngülle (sogenannte Separatorflüssigkeit bzw. Separatorfluid (55) s.o.) und Weizenstroh (gehäckselt in verschiedenen Größen) in einem Mischungsverhältnis von 4 : 1 bei einem TS Wert von ca. 25 bei verschiedenen Temperaturen. Das Verfahren gemäß der vorliegenden Erfindung (G9-S3, G11-S4, G17-S7 und G19-S8) zeigte im Vergleich zu dem Verfahren, das gemäß dem Stand der Technik ohne Aktivatorvorrichtung 21 durchgeführt wurde (G5-S1, G7-S2, G13-S5, G15-S6) eine deutlich erhöhte Menge an erhaltenem Biogas, wobei am meisten Biogas bei einer Temperatur von 63°C erhalten wurde (G19-S8). Das Verfahren wurde in einer Anlage 60 gemäß Figur 5 durchgeführt, wobei wie oben schon gesagt, beim Verfahren des Standes der Technik keine Aktivatorvorrichtung 21 in der Anlage vorhanden war.

### Bezugszeichen

Bezugzeichenliste für Figur 5
- 1: Endlagerbehälter
- 2: Separator
- 3: Separator Fluidbehälter
- 4: Abscheider
- 5: Fäller
- 6: Bionik HAT Tank
- 7: Bionik LT Tank
- 8: Feststoffmischer
- 9: Feststoffeintrag
- 21: Rekuperator Wärmetauscher
- 22: Vorerhitzer Wärmetauscher
- 23: Nacherhitzer Wärmetauscher
- 31: Druck Pumpe 4 bar 20-50 °C
- 32: Zirkulationspumpe 1 bar 70-90 °C
- 41: Zirkulations Ventil im Vorheizkreis
- 42: Dosier Ventil zum Feststoffeintrag
- 43: Einlauf Ventil zum Vorerhitzer
- 44: Überlauf Ventil vom Vorerhitzer in Separatorfluid
- 45: Ablauf Ventil vom Abscheider
- 51: Separator Material Feststoff Gärrest
- 52: Separator Fluid TS04 kalt dünnflüssig
- 53: Separatorfluid im Außenmantel aufgewärmt
- 54: Separatorfluid durch Einlaufventil 50 °C
- 55: Separatorfluid TS04 im Vorerhitzerkreis 80 °C
- 56: E-Mix nach Zugabe von Feststoffen <70 °C
- 57: E-Mix TS12 nach Erhitzung 80 °C
- 58: E-Mix TS12 abgekühlt 55 °C zum Fermenter
- 91: Motorkühlwasser heizt Vorerhitzer 22
- 92: Motorkühlwasser heizt Nacherhitzer 23
- 93: Motorkühlwasser heizt Separatorfluid 3 bei Bedarf
- 94: Motorkühlwasser heizt Bionik HT Tank 6
- 95: Motorkühlwasser heizt Feststoffeintrag bei Bedarf

Die Betriebsweise des BMT Systems ist bevorzugt getaktet.
A. Einlasstakt und zugleich Ausstoß aus der Zirkulation in die Zumischung
B. Erhitzungstakt in Zirkulation und zugleich Nacherhitzung im Wärmetauscher 23 sowie Kühlung in WT 21. Dann wieder A.

## Patentansprüche

1. Verfahren zur Erzeugung einer Biomassezubereitung, umfassend die Schritte des:
a) Bereitstellens eines schwer aufschließbaren ligninhaltigen biologischen Edukts,
b) Bereitstellens eines mikrobiologischen Aktivators zum Aufschließen des Edukts, der bei Überschreiten einer Temperatur T_{A} aktivierbar ist und
c) Aktivierens einer Kombination aus Edukt und mikrobiologischen Aktivator zum Aufschließen des Edukts mittels einer Einrichtung zur thermischen Aktivierung,
**dadurch gekennzeichnet, dass** der mikrobiologische Aktivator 1 Million thermophile Mikroben pro Milliliter umfasst,
wobei zumindest ein Anteil dieser Mikroben Lösemittelbildner ist,
wobei T_{A} > 70°C beträgt.

2. Verfahren nach Anspruch 1, wobei das Edukt ligninhaltig ist.

3. Verfahren nach Anspruch 1 oder 2, wobei das Edukt fest ist oder vor Schritt a) mechanisch zerkleinert wird.

4. Verfahren nach Anspruch 3, wobei das Edukt nach Schritt a) oder b) mit einer Flüssigkeit vermischt wird.

5. Verfahren nach Anspruch 4, wobei die Mischung auf eine Temperatur T₁ gebracht wird, wobei T₁ < T_{A}.

6. Verfahren nach Anspruch 4 oder 5, wobei die Mischung auf einen TS Wert zwischen 9 und 15 eingestellt wird.

7. Verfahren nach Anspruch 6, wobei die Mischung auf einen TS Wert zwischen 10 und 15 eingestellt wird.

8. Verfahren nach einem der vorhergehenden Ansprüche, wobei nach Schritt c) die Reaktionsmischung einem Fermenter zugeführt wird.

9. Verfahren nach Anspruch 8, wobei die Reaktionsmischung vor der Zuführung in den Fermenter auf eine Temperatur T_{F} mit T_{F} < T_{A} abgekühlt wird.

10. Verfahren nach einem der vorhergehenden Ansprüche, wobei vor oder während dem Aktivieren die Kombination aus Edukt und Aktivator auf einen Druck größer als ein Aktivierungsdruck komprimiert wird, wobei der Aktivierungsdruck größer als 1,3 bar ist.

11. Verfahren nach einem der vorhergehenden Ansprüche, wobei das Aktivieren bewirkt, dass mindestens eine mikrobiologische Komponente beginnt, Ligninschichten abzulösen und anschließend mindestens eine mikrobiologische Komponente beginnt, Ligninfragmente aufzuschließen.

12. Verfahren nach einem der vorhergehenden Ansprüche wobei das im Fermenter entstehende Biogas abgetrennt wird.

13. Vorrichtung (10, 20, 30, 40, 60) zur Verwendung für die Durchführung des Verfahrens nach einem der Ansprüche 1 bis 12 zur Erzeugung einer Biomassezubereitung, umfassend:
- eine Einrichtung (9, 31, 41) zum Einbringen eines schwer aufschließbaren ligninhaltigen biologischen Edukts in die Vorrichtung (10, 20, 30, 40, 60),
- eine Aktivatorvorrichtung (21) mit einem mikrobiologischen Aktivator zum Aufschließen des Edukts, der bei Überschreiten einer Temperatur T_{A} aktivierbar ist und mit einer Vorrichtung zur thermischen Aktivierung der Aktivatorvorrichtung (21),
**dadurch gekennzeichnet, dass** der mikrobiologische Aktivator 1 Million thermophile Mirkoben pro Milliliter umfasst,
wobei zumindest ein Anteil dieser Mikroben Lösemittelbildner ist,
wobei T_{A} > 70°C beträgt.

14. Vorrichtung (10, 20, 30, 40, 60) nach Anspruch 13, wobei das schwer aufschließbare biologische Edukt ligninhaltig ist.

15. Vorrichtung (10, 20, 30, 40, 60) nach einem der vorhergehenden Ansprüche, wobei die Aktivatorvorrichtung (21) einen Wärmetauscher oder eine Heizeinrichtung umfasst.

16. Vorrichtung (10, 20, 30, 40, 60) nach einem der vorhergehenden Ansprüche, weiterhin umfassend:
- eine Mischeinrichtung für Edukte.

17. Vorrichtung (10, 20, 30, 40, 60) nach einem der vorhergehenden Ansprüche, weiterhin umfassend:
- eine Dosiereinrichtung für eine dosierte Zugabe des Mittels zum Aufschließen des Edukts.

18. Vorrichtung (10, 20, 30, 40, 60) nach einem der vorhergehenden Ansprüche, weiterhin umfassend:
- eine Mischeinrichtung zum Mischen des Edukts aus verschiedenen festen, breiförmigen oder flüssigen Edukten.

19. Vorrichtung (10, 20, 30, 40, 60) nach einem der vorhergehenden Ansprüche, weiterhin umfassend:
- eine Heizeinrichtung, die erwärmt wird durch chemische Energie oder Reaktionswärme oder elektromagnetische Energie oder Strahlung.

20. Vorrichtung (10, 20, 30, 40, 60) nach einem der vorhergehenden Ansprüche, weiterhin umfassend:
- eine Einrichtung zur Wärmerückgewinnung.

## Claims

1. Process for the production of a biomass preparation, comprising the steps of:
a) providing a poorly digestible lignin-containing biological educt,
b) providing an microbiological activator for digesting the educt, which is activatable when a temperature T_{A} is exceeded, and
c) activating a combination of educt and microbiological activator for digesting the educt by means of a device for thermal activation,
**characterized in that** the microbiological activator comprises 1 million thermophilic microbes per millilitre,
wherein at least one portion of these microbes is solvent binder,
wherein T_{A} > 70°C.

2. Process according to Claim 1, wherein the educt is lignin-containing.

3. Process according to Claim 1 or 2, wherein the educt is solid or is mechanically comminuted before step a).

4. Process according to Claim 3, wherein the educt after step a) or b) is blended with a liquid.

5. Process according to Claim 4, wherein the mixture is brought to a temperature T₁, wherein T₁ < T_{A}.

6. Process according to Claim 4 or 5, wherein the mixture is adjusted to a DS value between 9 and 15.

7. Process according to Claim 6, wherein the mixture is adjusted to a DS value between 10 and 15.

8. Process according to one of the previous claims, wherein after step c) the reaction mixture is fed into a fermenter.

9. Process according to Claim 8, wherein before feeding into the fermenter the reaction mixture is cooled to a temperature T_{F} with T_{F}< T_{A}.

10. Process according to one of the previous claims, wherein before or during the activation, the combination of educt and activator is compressed to a pressure greater than an activation pressure, wherein the activation pressure is greater than 1.3 bar.

11. Process according to one of the previous claims, wherein the activation has the effect that at least one microbiological component begins to detach lignin layers and next at least one microbiological component begins to digest lignin fragments.

12. Process according to one of the previous claims wherein the biogas forming in the fermenter is separated.

13. Device (10, 20, 30, 40, 60) for use for carrying out the process according to one of Claims 1 to 12 for the production of a biomass preparation, comprising:
- a device (9, 31, 41) for introducing a poorly digestible lignin-containing biological educt into the device (10, 20, 30, 40, 60),
- an activator device (21) with a microbiological activator for digestion of the educt, which is activatable when a temperature T_{A} is exceeded, and with a device for the thermal activation of the activator device (21),
**characterized in that** the microbiological activator comprises 1 million thermophilic microbes per millilitre,
wherein at least one portion of these microbes is solvent binder,
wherein T_{A} > 70°C.

14. Device (10, 20, 30, 40, 60) according to Claim 13, wherein the poorly digestible biological educt is lignin-containing.

15. Device (10, 20, 30, 40, 60) according to one of the previous claims, wherein the activator device (21) comprises a heat exchanger or a heating device.

16. Device (10, 20, 30, 40, 60) according to one of the previous claims, further comprising:
- a mixing device for educts.

17. Device (10, 20, 30, 40, 60) according to one of the previous claims, further comprising:
- a metering device for metered addition of the agent for digestion of the educt.

18. Device (10, 20, 30, 40, 60) according to one of the previous claims, further comprising:
- a mixing device for mixing the educt consisting of different solid, slurry or liquid educts.

19. Device (10, 20, 30, 40, 60) according to one of the previous claims, further comprising:
- a heating device which is heated by chemical energy or heat of reaction or electromagnetic energy or irradiation.

20. Device (10, 20, 30, 40, 60) according to one of the previous claims, further comprising:
- a device for heat recovery.

## Revendications

1. Procédé de production d'une préparation de biomasse comprenant les étapes de
a) mettre & disposition un matériau de départ biologique peu digestible contenant de la lignine,
b) mettre & disposition un activateur microbiologique qui est activable lorsqu'une température T_{A} est dépassée pour désintégrer le matériau de départ, et
c) activer une combinaison de matériau de départ et d'activateur microbiologique pour digérer le matériau de départ au moyen d'un dispositif d'activation thermique,
**caractérisé en ce que** l'activateur microbiologique comprend 1 million de microbes thermophiles par millilitre,
dans lequel au moins une partie de ces microbes sont des formateurs de solvant,
dans lequel T_{A} est > 70°C

2. Procédé selon la revendication 1, dans lequel le matériau de départ comprend de la lignine.

3. Procédé selon la revendication 1 ou 2, dans lequel le matériau de départ est solide ou est broyé mécaniquement avant l'étape a).

4. Procédé selon la revendication 3, dans lequel le matériau de départ est mélangé avec un liquide après l'étape a) ou b).

5. Procédé selon la revendication 4, dans lequel le mélange est porté à une température T₁, dans lequel T₁ <T_{A}.

6. Procédé selon la revendication 4 ou 5, dans lequel le mélange est ajusté à une valeur TS comprise entre 9 et 15.

7. Procédé selon la revendication 6, dans lequel le mélange est ajusté à une valeur TS comprise entre 10 et 15.

8. Procédé selon l'une des revendications précédentes, dans lequel, après l'étape c), le mélange réactionnel est introduit dans un fermenteur.

9. Procédé selon la revendication 8, dans lequel le mélange réactionnel est refroidi à une température T_{F} avec T_{F} <T_{A} avant d'être introduit dans le fermenteur.

10. Procédé selon l'une des revendications précédentes, dans lequel avant ou pendant l'activation la combinaison de matériau de départ et de l'activateur est comprimée à une pression supérieure à une pression d'activation, dans laquelle la pression d'activation est supérieure à 1,3 bar.

11. Procédé selon l'une des revendications précédentes, dans lequel l'activation provoque qu'au moins un composant microbiologique commence à détacher des couches de lignine et ensuite au moins un composant microbiologique commence à désintégrer des fragments de lignine.

12. Procédé selon l'une des revendications précédentes, dans lequel le biogaz produit dans le fermenteur est séparé.

13. Dispositif (10, 20, 30, 40, 60) pour être utilisé pour la mise en œuvre du procédé selon l'une des revendications 1 à 12 pour la production d'une préparation de biomasse, comprenant:
- un dispositif (9, 31, 41) pour introduire un matériau de départ biologique contenant de la lignine peu digestible dans le dispositif (10, 20, 30, 40, 60),
- un dispositif activateur (21) ayant un activateur microbiologique pour digérer le matériau de départ pouvant être activé lorsqu'une température TA est dépassée, et ayant un dispositif pour l'activation thermique du dispositif activateur (21),
**caractérisé en ce que** l'activateur microbiologique comprend 1 million de microbes thermophiles par millilitre,
dans lequel au moins une partie de ces microbes sont des formateurs de solvant,
dans lequel T_{A} est > 70°C.

14. Dispositif (10, 20, 30, 40, 60) selon la revendication 13, dans lequel le matériau de départ biologique peu digestible comprend de la lignine.

15. Dispositif (10, 20, 30, 40, 60) selon l'une des revendications précédentes, dans lequel le dispositif activateur (21) comprend un échangeur de chaleur ou un dispositif de chauffage.

16. Dispositif (10, 20, 30, 40, 60) selon l'une des revendications précédentes, comprenant en outre:
- un dispositif de mélange pour des matériaux de départ.

17. Dispositif (10, 20, 30, 40, 60) selon l'une des revendications précédentes, comprenant en outre:
- un dispositif de dosage pour une addition dosée des moyens de digestion du matériau de départ.

18. Dispositif (10, 20, 30, 40, 60) selon l'une des revendications précédentes, comprenant en outre:
- un dispositif de mélange pour mélanger le matériau de départ à partir de divers matériaux de départ solides, bouillis ou liquides.

19. Dispositif (10, 20, 30, 40, 60) selon l'une des revendications précédentes, comprenant en outre:
- un dispositif de chauffage chauffé par une énergie chimique ou une chaleur de réaction ou une énergie électromagnétique ou un rayonnement.

20. Dispositif (10, 20, 30, 40, 60) selon l'une des revendications précédentes, comprenant en outre:
- un appareil pour la récupération de chaleur.
